# EUROPÄISCHE PATENTSCHRIFT

(11) **EP 1 746 083 B1**
(45) Veröffentlichungstag und Bekanntmachung des Hinweises auf die Patenterteilung: **21.12.2016**
(21) Anmeldenummer: 06013434.3
(22) Anmeldetag: 29.06.2006
(51) Int. Cl.: C07C 209/36, C07C 209/84, C07C 211/50, C07C 263/10, C07C 265/12

(54) **Verfahren zur Herstellung von Toluylendiamin**
Process for the production of toluylene diamine
Procédé de production de toluylène diamine

(30) Priorität: 12.07.2005 DE 10532430
(43) Veröffentlichungstag der Anmeldung: 24.01.2007
(73) Patentinhaber: Covestro Deutschland AG, 51373 Leverkusen (DE)
(72) Erfinder: Pennemann, Bernd, Dr., 51467 Bergisch Gladbach (DE); Brady, Bill, Houston 77059 (DE); Buse, Rainer, Dr., 51061 Köln (DE); Greger, Gerd, 47906 Kempen (DE)
(74) Vertreter: Levpat

(56) Entgegenhaltungen:
- EP-A1- 0 757 034
- WO-A-02/48075
- WO-A-2005/066113

## Beschreibung

Die Erfindung betrifft ein Verfahren zur Herstellung von Toluylendiamin (TDA) und dessen Auftrennung in erwünschte und unerwünschte Komponenten, wobei Dinitrotoluol hydriert und vom so erhaltenen Reaktionsgemisch ggf. vorhandenes Lösungsmittel sowie Wasser und andere Nebenprodukte durch Destillation abgetrennt werden und für die anschließende Auftrennung des entwässerten Gemisches in erwünschte Isomeren sowie unerwünschte Isomeren, noch vorhandene Nebenprodukte und hochsiedende Verbindungen eine Trennwandkolonne eingesetzt wird.

Nach dem Stand der Technik wird m-TDA (ein 2,4- und 2,6-Isomerengemisch) für den Einsatz in der Phosgenierung zu TDI durch die Hydrierung von Dinitrotoluol hergestellt. Die Hydrierung kann dabei in Gegenwart von Lösungsmitteln erfolgen. Üblicherweise wird zunächst der Katalysator abgetrennt. Dies kann beispielsweise durch Filtration oder Sedimentation geschehen. Bei der Hydrierung entstehen neben dem Zielprodukt TDA und dem Nebenprodukt Wasser auch organische Nebenprodukte. Hierzu gehören Leichtsieder und Hochsieder. Bei Leichtsiedern handelt es sich um Verbindungen, die einen Siedepunkt haben, der unterhalb dem Siedepunkt von TDA liegt. Bei Hochsiedern handelt es sich um Verbindungen, die einen höheren Siedepunkt als das Zielprodukt TDA haben.

Diese Nebenprodukte können den Einsatz des Zielprodukts TDA in den Anwendungen, besonders der Phosgenierung zu Toluylendiisocyanat (TDI) empfindlich stören. Daraus ergibt sich die Notwendigkeit, das erhaltene Reaktionsgemisch in seine Komponenten zu trennen.

Wird Lösungsmittel in der Hydrierreaktion zugesetzt, wird dieses zunächst abgetrennt. Dies geschieht im allgemeinen in bekannter Weise durch Destillation in einer kontinuierlich betriebenen Destillationskolonne, wobei das Lösungsmittel durch geeignete Verfahrensführung so rein zurückerhalten wird, dass es ohne weitere Reinigung direkt im Prozeß verwendet werden kann. Es ist auch möglich, das Lösungsmittel gemeinsam mit einem Teil oder der gesamten Menge des gebildeten Wassers aus dem Reaktionsprodukt destillativ abzutrennen und das Lösungsmittel daran anschließend in einer weiteren Verfahrensstufe in der benötigten Reinheit zurückzugewinnen. Bei Durchführung der Reaktion ohne Lösungsmittelzusatz ist diese Lösungsmittelabtrennung selbstverständlich nicht nötig.

Es schließt sich dann üblicherweise die eigentliche TDA-Trocknung an, d.h. die Entfernung des Reaktionswassers, das ca. 40 Gew-% des erhaltenen Reaktionsgemisches ausmacht. Dies kann prinzipiell durch einfaches Abtreiben des Wassers durch Erhitzen der TDA-Lösung im Vakuum und Abziehen der Dämpfe erfolgen. Jedoch fällt bei dieser einfachen Verfahrensführung das abgetrennte Wasser nicht in der für eine problemlose Entsorgung erforderlichen Reinheit an, sondern ist stets mit TDA verunreinigt. Das Reaktionswasser wird daher besser durch Destillation in einer geeigneten Destillationsapparatur entfernt. Dazu wird z.B. die TDA-Rohlösung in einer Kolonne bis auf eine Temperatur von 200°C erhitzt, wobei das Wasser am Kopf in reiner Form anfällt, wenn die Kolonne bei Normaldruck oder leichtem Überdruckbereich betrieben wird und etwa 20 bis 30 Böden besitzt. Das TDA wird dann aus dem Sumpf abgezogen und durch Entspannung ins Vakuum von 30 bis 300 mbar von letzten Wasserspuren befreit.

Eine Variante dieses Verfahrens wird in EP-A-0236 839 beschrieben und erlaubt die Abtrennung von Wasser und leichtflüchtigen Verbindungen und teilweise auch Leichtsiedern.

Das erhaltene Gemisch (Roh-TDA) besteht üblicherweise aus m-TDA mit weniger als 10 Gew.-% ortho-Isomeren, weniger als 5 Gew.-% Hochsiedern, weniger als 5 Gew.-% Leichtsiedern und weniger als 5 Gew-% Wasser. Bekannt ist die Abtrennung der 2,3- und 3,4-Isomeren aus dem TDA-Isomerengemisch. So zeigt beispielsweise US-A-3,420,752 den Einsatz einer Destillationskolonne für diese Trennung. Dabei wird am Kopf o-TDA und im Sumpf m-TDA erhalten. Da das aus der Reaktion erhaltene Roh-TDA regelmäßig Leichtsieder und Hochsieder enthält, ist es unmittelbar einsichtig, dass ohne weitere Maßnahmen das Kopfprodukt nicht nur o-TDA, sondern auch Leichtsieder enthält. Ebenso wird das Sumpfprodukt nicht nur m-TDA sondern auch Hochsieder enthalten. Im Falle des m-TDA stellen diese Verunreinigungen einen Nachteil dieses Verfahrens dar, denn diese Hochsieder sind im Zielprodukt unerwünscht und man ist daher bestrebt, diese vor dem weiteren Einsatz des m-TDA, beispielsweise in der Phosgenierung zu TDI, abzutrennen.

WO-A-02/48075 beschreibt ein Verfahren zur destillativen Trennung von m- und o-TDA Isomeren.

US-A-6,547,933 beschreibt hierzu ein Verfahren, bei dem ein von Hochsiedern befreites m-TDA erhalten wird. Eine Variante des Verfahrens sieht dabei die Trennung des roh-TDA in seine Isomeren in einer Destillationskolonne vor, wobei im unteren Drittel der Destillationskolonne ein Teilstrom als Brüden entnommen und auf einen Kondensator gegeben wird. Auf diese Weise wird von Hochsiedern befreites m-TDA erhalten. Ein erheblicher Teil des Produktstroms besteht jedoch nach wie vor aus m-TDA mit Anteilen von Hochsiedern, die beispielsweise dann in einer nachgeschalteten Phosgenierung eingesetzt werden. In einer weiteren Variante des Verfahrens wird ein m-TDA Strom aus der Kolonne entnommen und teilweise verdampft. Der so erhaltene Brüdenstrom wird kondensiert und auf diese Weise wird von Hochsiedern befreites m-TDA gewonnen. Der nicht verdampfte Anteil, der Hochsieder enthält, wird auf die Kolonne zurückgeführt. Im Sumpf der Kolonne wird ein weiterer m-TDA-Strom entnommen, der wiederum Hochsieder enthält. In beiden Varianten wird der im Sumpf ausgeschleuste und Hochsieder enthaltende Strom einen wesentlichen Teil des hergestellten m-TDA enthalten. Ein wirtschaftlicher Einsatz des Verfahrens ist also nur möglich, wenn dieser hochsiederhaltige Strom einer Nutzung zugeführt wird. Das Ziel der vorliegenden Erfindung wird auf diese Weise also nicht erreicht.

Das in US-A-6,359,177 dargestellten Verfahren führt zu einer vollständigen Abtrennung der Hochsieder vom m-TDA. Hierzu wird zunächst das TDA in einer Destillationskolonne in seine Isomeren getrennt. Das als Sumpfprodukt erhaltene Gemisch aus m-TDA und Hochsiedern wird in einem zweiten Apparat bestehend aus Verdampfer und Kondensator in einen m-TDA-Strom und einen Hochsiederstrom aufgetrennt. Das in dem Hochsiederstrom noch enthaltene m-TDA wird in einer weiteren Strippkolonne abgereichert und teilweise durch o-TDA ersetzt. Dabei wird ein Strom erhalten, der im wesentlichen Hochsieder und o-TDA enthält und ausgeschleust und beispielsweise verbrannt wird. Ein zweiter Strom besteht aus o-TDA und m-TDA, dieser Strom wird auf die Isomerendestillationskolonne zurückgeführt. Varianten dieses Verfahrens werden ebenfalls in US-A-6,359,177 beschrieben. Dieses Verfahren erreicht das Ziel einer Minimierung der m-TDA-Verluste in dem auszuschleusenden Hochsiederstrom. Es erfordert jedoch einen Mehraufwand an Apparaten und Energie.

In der thermischen Trenntechnik ist es oft erwünscht, ein Mehrkomponentengemisch in seine Einzelkomponenten aufzutrennen. Im Falle eines Zulaufs und zwei Produktströmen können Kopf- und Sumpfablauf einer Destillationskolonne verwendet werden. Bei Mehrkomponentengemischen kann eine weitere Aufteilung durch Wiederholung der Trennung in zwei Ströme erreicht werden, Nachteilig ist, dass dieses Vorgehen zusätzliche Apparate wie Kolonnen, Kondensatoren oder Verdampfer erfordert. Dies wiederum erhöht die für den Betrieb erforderlichen Energiemengen sowie die damit verbundenen Kosten. Zahlreiche Veröffentlichungen befassen sich mit der Aufgabe, den mit der Trennung eines Stoffgemisches erforderlichen apparativen und energetischen Aufwand zu senken. Dabei stellt das System von Petlyuk (siehe z.B. R. Agrawal, Z. Fidowski, "Are Thermally coupled distillation columns always thermo-dynamically more efficient for ternary distillations?", Ind. Eng. Chem. Res., 1998, 37 pp- 3444-3454) den Maßstab für die energetische Effizienz einer Trennsequenz dar. Bei dieser Konfiguration trennt eine Vortrennkolonne den Zulauf in zwei Ströme unter Einsatz des aufgeteilten Brüdenstroms des Abtriebteils der Hauptkolonne und des geteilten Flüssigkeitsablaufs des Verstärkerteils der Hauptkolonne. Die erhaltenen Dampf- und Flüssigkeitsströme, die die Vortrennkolonne verlassen, sind an Leicht- bzw. Schwersiedern angereichert. Diese beiden Ströme werden in die Hauptkolonne geführt. Diese Konfiguration bietet Vorteile hinsichtlich der Reinheit des als Seitenstrom entnommenen Produkts. Andererseits wird durch diese Anordnung die Reinheit der Zuläufe in den Abtriebs- und Verstärkerteil der Hauptkolonne verbessert. Auf diese Weise wird eine hohe Reinheit der drei Produktströme erreicht.

US-A-2,471,134 stellt eine Verbesserung dieses Vorgehens dar, in dem die Vortrenn- und die Hauptkolonne in einem Apparat vereinigt werden, der in der Mitte durch eine Trennwand geteilt wird. Diese Kolonne ist mit einem Verdampfer und einem Kondensator ausgestattet. Die Kolonne besteht dann aus 4 Segmenten. Diese sind ein gemeinsamer Verstärkerteil am Kopf der Kolonne, ein gemeinsamer Abtriebsteil am Sumpf der Kolonne sowie ein Vortrenn- und ein Hauptsegment, die sich im Mittelteil der Kolonne nebeneinander befinden und durch eine Wand getrennt sind. Das Gemisch wird auf oder in das Vortrennsegment gegeben, das Kopfprodukt wird oberhalb des gemeinsamen Verstärkerteils abgezogen und das Sumpfprodukt unterhalb des gemeinsamen Abtriebteils. Das zwischensiedende Produkt wird als Seitenstrom dem Hauptsegment entnommen. Diese Trennwandkolonne bietet Vorteile hinsichtlich der Hydraulik des Gesamtsystems und verringert die Apparatekosten des Verfahrens nach Petlyuk.

Die Aufgabe der vorliegenden Erfindung besteht darin, ein einfaches und wirtschaftliches Verfahren zur Herstellung von Toluylendiamin und dessen Auftrennung in m-TDA, o-TDA, Leichtsieder und Hochsieder (Schwersieder) zur Verfügung zu stellen, bei dem das im Roh-TDA enthaltene m-TDA von o-TDA, Leichtsiedern und Hochsiedern befreit wird und gleichzeitig die Verluste an m-TDA sowie der apparative Aufwand und die für das Verfahren erforderliche Energiemenge gering gehalten wird.

Die Erfindung betrifft ein Verfahren zur Herstellung von m-TDA gemäß Patentanspruch 1, bei dem
a) Dinitrotoluol in Gegenwart eines Katalysators hydriert wird und anschließend Katalysator, Wasser und gegebenenfalls Lösungsmittel abgetrennt wird, wobei Roh-Toluylendiamin erhalten wird, und
b) das Roh-Toluylendiamin in einer Trennwandkolonne destillativ getrennt wird, wobei wenigstens vier Produktströme P1 - P4 erhalten werden, wobei
   der Produktstrom P1 ein Leichtsieder enthaltender Strom ist, und
   der Produktstrom P2 ein o-TDA enthaltender Strom ist, und
   der Produktstrom P3 ein m-TDA enthaltender Strom ist, und
   der Produktstrom P4 ein Hochsieder und m-TDA enthaltender Strom ist, und bei dem das Roh-Toluylendiamin der Trennwandkolonne seitlich von der Trennwand (8) zugeführt wird, wobei die Aufgabe des Roh-Toluylendiamin von oben auf ein Trennsegment (4a) erfolgt, dessen Oberkante mindestens 10 % der Gesamthöhe der Trennwand unterhalb der Oberkante der Trennwand liegt und mindestens 10 % der Gesamthöhe der Trennwand oberhalb der Unterkante der Trennwand liegt.

Unter dem Begriff m-TDA sind die Isomeren 2,4-TDA und 2,6-TDA zusammengefasst. Unter dem Begriff o-TDA sind entsprechend die Isomeren 2,3-TDA und 3,4-TDA zusammengefasst.

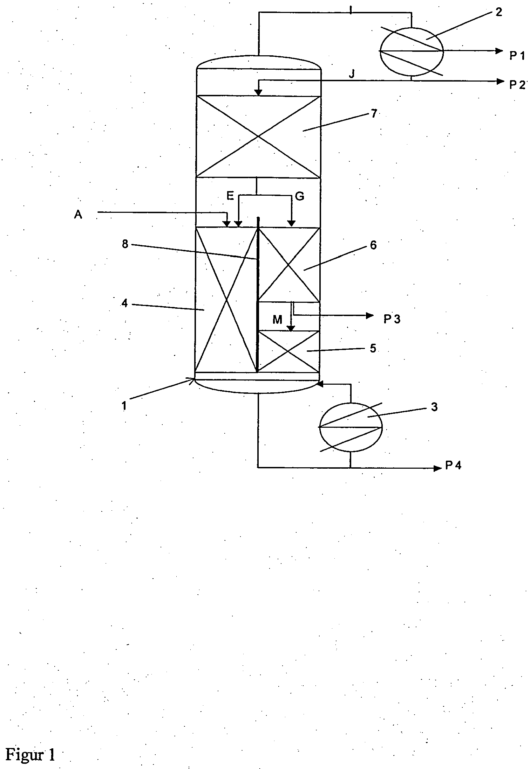

Die Aufgabe wird gelöst durch den Einsatz einer Trennwandkolonne in dem erfindungsgemäßen Verfahren. Figuren 1 bis 5 zeigen Varianten von Trennwandkolonnen, die in dem erfindungsgemäßen Verfahren eingesetzt werden können.

Der Zulauf der Trennwandkolonne (Zulauf A in den Figuren 1 - 5) enthält im wesentlichen (d.h. bevorzugt zu mindestens 75 Gew.%, besonders bevorzugt zu mindestens 87 Gew.-%, ganz besonders bevorzugt zu mindestens 93 Gew.-%) m-TDA und enthält bevorzugt zusätzlich weniger als 10 Gew.-% o-TDA, weniger als 5% Gew. Hochsieder, weniger als 5 Gew.-% Leichtsieder sowie weniger als 5 Gew.-% Wasser. Besonders bevorzugt enthält der Zulauf zusätzlich zum m-TDA weniger als 3 Gew.-% Hochsieder, weniger als 2 Gew,-% Leichtsieder, weniger als 2 Gew.-% Wasser und weniger als 6 Gew.-% o-TDA. Ganz besonders bevorzugt enthält der Zulauf zusätzlich zum m-TDA weniger als 2 Gew.-% Hochsieder, weniger als 1 Gew.-% Leichtsieder, weniger als 1 Gew.-% Wasser und zwischen 2 und 5 Gew.-% o-TDA.

Produktstrom P1 ist ein Leichtsieder enthaltender Brüdenstrom, der bevorzugt Inerte, Wasser, Leichtsieder und o-TDA enthält. Inerte sind beispielsweise Stickstoff oder Luft, Leichtsieder sind beispielsweise Toludine und Diaminomethylcyclohexan. Bevorzugt beträgt der Gehalt an o-TDA in diesem Strom weniger als 75 Gew.-% und besonders bevorzugt weniger als 50 Gew.-%: Die Menge an Wasser entspricht dabei im wesentlichen der Menge im Zulauf A, der Rest des Stroms sind im wesentlichen Leichtsieder. Bevorzugt enthält dieser Strom weniger als 30 Gew.-% Wasser. Um eine nachgeschaltete Vakuumerzeugung nicht unnötig zu belasten, kann dieser Strom einer Nachkondensation unterzogen werden, so dass insbesondere die Leichtsieder aufgefangen und entsorgt werden können.

Der Produktstrom P2 enthält bevorzugt überwiegend o-TDA und kann daneben auch Leichtsieder und m-TDA enthalten. Der Gehalt an m-TDA ist dabei in weiten Grenzen einstellbar. In der Praxis wird man ohne zwingenden Grund 20 Gew.-% m-TDA jedoch selten überschreiten. Bevorzugt sind Konzentrationen unter 10 Gew.% m-TDA und besonders bevorzugt sind unter 5 Gew.-%. Ob die Ströme P1 und P2 gemeinsam anfallen oder getrennt werden müssen, hängt von der geplanten Verwendung des o-TDA ab. Wie gegebenenfalls diese Trennung erfolgt, hängt von der Zusammensetzung des Zulaufs sowie der geforderten Reinheit des o-TDA ab. Sofern eine Trennung erforderlich ist, wird man in der Praxis regelmäßig weniger als 5 Gew.-% Leichtsieder erhalten wollen, bevorzugt weniger als 3 Gew.-% und besonders bevorzugt weniger als 1 Gew.-%. Bevorzugt ist ein Gehalt an o-TDA von mehr als 90 Gew.-% und besonders bevorzugt ein Gehalt von mehr als 97 Gew.-%.

Der Produktstrom P3 enthält bevorzugt überwiegend m-TDA. Abhängig von der Zahl der theoretischen Stufen der Trennwandkolonne und/oder des Rücklaufverhältnisses kann der Gehalt an o-TDA eingestellt werden. Bevorzugt sind Gehalte an o-TDA von unter 2 Gew.-%, besonders bevorzugt von unter 1 Gew.-% und ganz besonders bevorzugt unter 0,5 Gew.-%. Der Gehalt an Hochsiedern beträgt bevorzugt weniger als 0,5 Gew.-% und besonders bevorzugt weniger als 0,3 Gew.-%.Bevorzugt ist ein m-TDA-Gehalt von mehr als 97 Gew.-%, besonders bevorzugt von mehr als 98,5 Gew.-% und ganz besonders bevorzugt von mehr als 99,5 Gew.-%.

Der Produktstrom P4 ist ein Hochsieder und m-TDA enthaltender Strom. Bevorzugt beträgt der Gehalt an m-TDA im Produktstrom P4 20 bis 80 Gew.-%, besonders bevorzugt 30-70 Gew.-%. Der Gehalt an Hochsiedern beträgt bevorzugt mehr als 20 Gew.-%, besonders bevorzugt mehr als 30 Gew.-% und ganz besonders bevorzugt mehr als 40 Gew.-%, aber weniger als 80 Gew.-%.

Bevorzugt wird der Produktstrom P4 weiter behandelt, um das darin enthaltene m-TDA zurückzugewinnen.

Die Erfindung wird nachfolgend anhand der Figuren näher erläutert. Es zeigen:
- Fig. 1: zeigt eine schematische Darstellung einer Trennwandkolonne in dem erfindungsgemäßen Verfahren.
- Fig. 2: zeigt eine alternative Ausführungsform des erfindungsgemäßen Verfahrens, bei dem ein zusätzlicher Kondensator eingesetzt wird. In diesem Fall stellt P5 einen, im Vergleich zum Brüdenstrom I aus der Kolonne 1 an Leichtsiedern angereicherten Strom dar, während P2 ein an Leichtsiedern abgereicherter Strom ist.
- Fig. 3: zeigt eine weitere alternative Ausführungsform des erfindungsgemäßen Verfahrens. In dieser Variante wird der o-TDA enthaltende Produktstrom P2 in hoher Reinheit durch Seitenentnahme aus der Trennwandkolonne erhalten.
- Fig. 4: zeigt eine weitere alternative Ausführungsform des erfindungsgemäßen Verfahrens, bei der Hochsieder und m-TDA enthaltende Strom P4 in einem weiteren Verfahrensschritt um m-TDA abgereichert wird.
- Fig. 5: zeigt eine weitere alternative Ausführungsform des erfindungsgemäßen Verfahrens, bei dem das Trennsegment zur Vortrennung des Zulaufs in zwei übereinander angeordnete Teilsegmente unterteilt ist und der Zulauf zwischen den beiden Teilsegmenten auf die Trennwandkolonne aufgegeben wird.

**Figur 1** zeigt eine schematische Darstellung einer Trennwandkolonne 1 in dem erfindungsgemäßen Verfahren. A stellt den Zulauf ("Roh-TDA") dar. Produktstrom P1 ist der Leichtsieder enthaltende Strom, Produktstrom P2 der o-TDA enthaltende Strom, Produktstrom P3 der m-TDA enthaltende Strom und Produktstrom P4 der Hochsieder und m-TDA enthaltende Strom. Die Trennwandkolonne 1 ist mit einem Kondensator 2, einem Verdampfer 3, einer Trennwand 8 sowie Trennsegment 4, 5, 6 und 7 versehen. Der Zulauf A wird dabei von oben auf das Trennsegment 4 aufgegeben. Trennsegment 4 dient der Vortrennung des Zulaufs A, Trennsegment 5 der Trennung von Hochsiedern und m-TDA. Dabei kann auch ein zusätzliches Trennsegment unterhalb der Trennwand vorgesehen werden. Der aus dem Segment 7 austretende Flüssigkeitsstrom wird aufgeteilt, Strom E wird auf Segment 4 und Strom G auf Segment 6 geleitet, das auf der entgegen gesetzten Seite der Trennwand 8 angeordnet ist. Der aus Segment 6 austretende Flüssigkeitsstrom wird geteilt, ein Teil wird als Strom M auf das darunter angeordnete Segment 5 geleitet, der Rest als Produkt P3 ausgeschleust.

Die Aufgabe des Roh-TDA (Strom A) erfolgt üblicherweise auf der einen Seite der Trennwand, die Entnahme des m-TDA enthaltenden Stroms P3 auf der anderen Seite der Trennwand und die Entnahme des Hochsieder und m-TDA enthaltenden Stroms P4 unterhalb der Trennwand. In einer erfindungsgemäßen Variante des Verfahrens wird der am Kopf der Kolonne erhaltene Brüdenstrom I, der im Wesentlichen o-TDA und Leichtsieder enthält, kondensiert und teilweise als Rücklauf J auf die Kolonne gegeben. Der nicht für den Rücklauf erforderliche Flüssigkeitsstrom wird als o-TDA enthaltender Strom P2 entnommen und einer weiteren Verwertung zugeführt.

Die Zahl der erforderlichen Trennstufen richtet sich nach den geforderten Reinheiten der Produktströme. Deren Bestimmung kann auf dem Fachmann bekannte Weise erfolgen. Bevorzugt haben die Segmente 4 und 6 mindestens 8 theoretische Böden und besonders bevorzugt zwischen 10 und 30. Das Segment 7 hat bevorzugt mindestens 13 theoretische Böden und ganz besonders bevorzugt zwischen 15 und 40. Wenn eine einstufige Trennung von m-TDA und Hochsiedern zur Einhaltung der gewünschten Zusammensetzung des Produktstroms P4 ausreichend ist, können Segment 5 und Strom M auch entfallen. Bevorzugt hat Segment 5 weniger als 10 theoretische Böden.

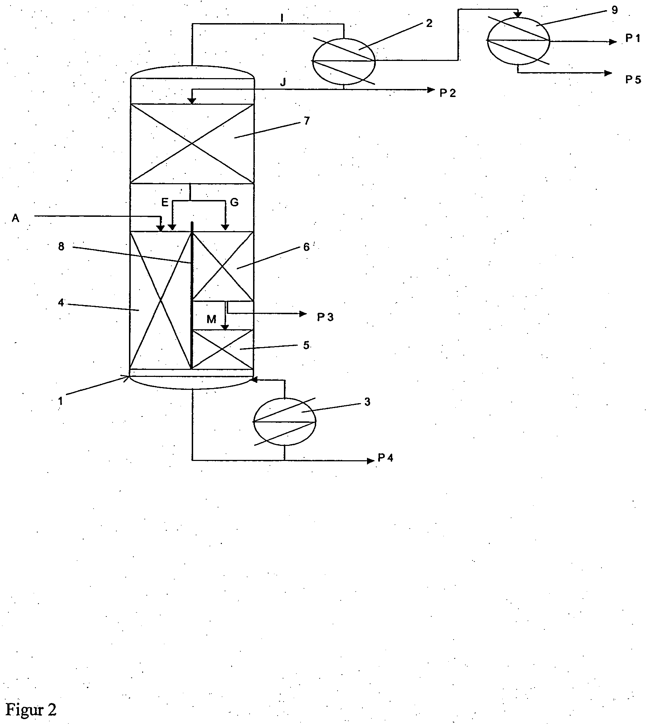

Wenn eine erhöhte Reinheit des o-TDA enthaltenden Stroms P2 erforderlich ist, kann in einer weiteren Variante des erfindungsgemäßen Verfahrens der am Kopf der Kolonne erhaltene Brüdenstrom I, der im Wesentlichen o-TDA und Leichtsieder enthält, im Kondensator 2 zunächst nur teilweise, d.h. üblicherweise nur zu 50 bis 90 Gew.-% der kondensierbaren Verbindungen, kondensiert werden. Diese Ausführungsform ist beispielhaft in Figur 2 gezeigt. Das so erhaltene Kondensat (Strom P2) weist einen höheren o-TDA-Gehalt auf als der Brüdenstrom I. Auf diese Weise wird ein reineres o-TDA erhalten, das dann teilweise als Rücklauf dient und als Produktstrom P2 ausgeschleust wird. Der im Kondensator 2 kondensierte Anteil richtet sich dabei nach dem Gehalt an o-TDA und Leichtsiedern im Zulauf sowie nach der geforderten Menge und Reinheit des o-TDA-Stromes P2. Die noch nicht kondensierten Brüden werden dabei üblicherweise in einem ein- oder mehrstufigen Kondensator 9 nachkondensiert. Die erhaltenen Kondensate (P5 in Figur 2 und ggf. weitere Kondensate) weisen dabei zunehmend höhere Leichtsiedergehalte auf und werden daher üblicherweise getrennt gesammelt und ihrer Verwertung zugeführt. Die Auslegung der Zahl der Kondensatoren und der jeweiligen Kondensationsleistung richtet sich dabei im wesentlich nach der geforderten Reinheit der o-TDA und Leichtsiederströme. Anhand dieser Informationen wird ein Fachmann leicht die günstigste Variante auswählen können. In dieser Ausführungsform stellt P5 einen, im Vergleich zum Brüdenstrom I aus der Trennwandkolonne 1 an Leichtsiedern angereicherten Strom dar, während P2 ein an Leichtsiedern abgereicherter Strom ist. Bevorzugt enthält Strom P5 weniger als 70 Gew.-% o-TDA, der Rest besteht im wesentlichen aus Leichtsiedern und Wasser.

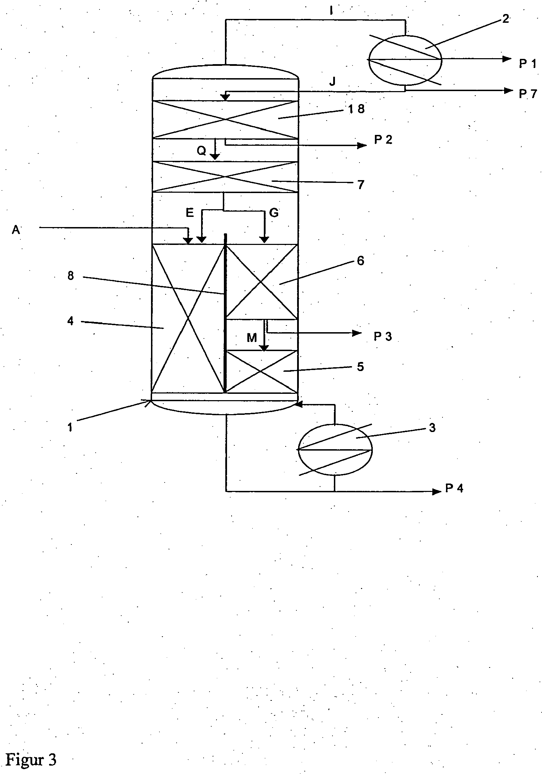

Eine andere bevorzugte Ausführungsform des erfindungsgemäßen Verfahrens führt ebenfalls zu höheren Reinheiten des Produktsstroms P2 an o-TDA bei gleichzeitiger Verringerung des in dem Leichtsieder enthaltenden Produktstroms P1 enthaltenen Anteils an o-TDA. Hierbei wird, wie in **Figur 3** dargestellt, die Destillationskolonne mit einer Seitenentnahme versehen, an der der o-TDA enthaltende Produktstrom P2 entnommen wird. Das oberhalb der Seitenentnahme angeordnete Trennsegment 18 wird dabei so ausgelegt, dass der gewünschte Gehalt an Leichtsiedern im o-TDA enthaltenden Produktstrom P2 erreicht werden kann. Strom Q ist der Rücklauf für das Segment 7. Bevorzugt wird man einen Gehalt von weniger als 3 Gew.-% Leichtsieder in Strom P2 einstellen, besonders bevorzugt sind weniger als 1 Gew.-%. Der Brüdenstrom I wird in dieser Ausführungform im Kondensator 2 kondensiert und das Kondensat teilweise als Rücklauf J auf die Kolonne gegebenen und teilweise als Strom P7 ausgeschleust. Die Zahl der Trennstufen des Segments 18 richtet sich nach der geforderten Reinheit des Produktstroms P2. Bevorzugt hat Segment 18 zwischen 1 und 20 theoretische Böden und ganz besonders bevorzugt zwischen 2 und 10.

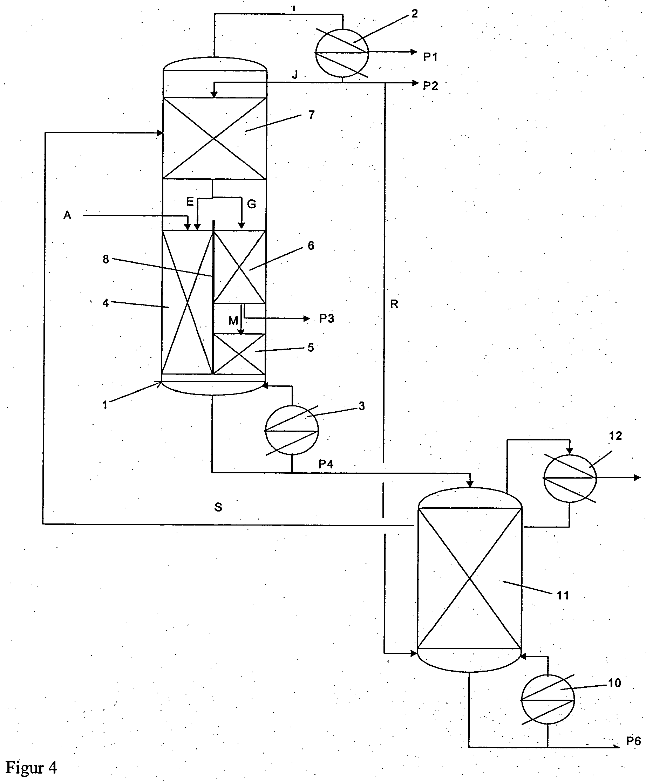

In einer weiteren Ausführungsform des erfmdungsgemäßen Verfahrens kann aus dem Hochsieder und m-TDA enthaltenden Produktstrom P4 in einem weiteren Verfahrensschritt m-TDA abgetrennt werden. Dazu wird der Hochsieder und m-TDA enthaltende Produktstrom P4 der Trennwandkolonne aus dem Sumpf entnommen und anschließend in einem zusätzlichen Apparat m-TDA abgetrennt. Dies kann auf verschiedene Weise erfolgen:
Die Isolierung des in dem Produktstrom P4 enthaltenen m-TDA und Abtrennung von den Hochsiedern kann in Anlehnung an EP-A-0794 170 oder US-A-6,359,177 erfolgen. Hierzu wird die Destillation des Roh-TDA in der Trennwandkolonne mit einer Rückgewinnung des im Gemisch aus Hochsiedern und m-TDA enthaltenen m-TDAs erfindungsgemäß kombiniert. Ein Beispiel einer bevorzugten Ausführungsform dieser neuen Kombination unter Verwendung der in US-A-6,359,177 offenbarten Technologie zeigt **Figur 4****.** Dabei wird der Strom P4 einem weiteren Verfahrensschritt, der in einem System enthaltend eine Destillationskolonne 11, einen Verdampfer 10 und einen Kondensator 12 durchgeführt wird, unterzogen. Dabei wird der flüssige Strom P4 der Destillationskolonne 11 am Kopf zugeführt. Der Strom P2 wird ganz oder teilweise (Strom R) unterhalb des Zulaufs des Stroms P4, beispielsweise in den Sumpf der Destillationskolonne 11, beispielsweise auch über den mit dem Sumpf der Destillationskolonne 11 hydraulisch verbundenen Verdampfer 10, aufgegeben. Aus dem Sumpf der Destillationskolonne 11, beispielsweise auch über den mit dem Sumpf der Destillationskolonne 11 hydraulisch verbundenen Verdampfer 10, wird ein Hochsieder, o-TDA und m-TDA enthaltender Strom P6 abgezogen. Der überwiegende Teil des im Zulauf P4 befindlichen m-TDA wird jedoch im Gemisch mit dem verbliebenen o-TDA als Strom S erhalten. Strom S wird zwecks weiterer Trennung auf die Trennwandkolonne 1 zurückgeführt. Auf diese Weise wird die zusammen mit den Hochsiedern ausgeschleuste Menge an m-TDA verringert und somit die Ausbeute des Verfahrens weiter gesteigert. Bevorzugt enthält der Strom P6 dabei zwischen 20 und 80 Gew.-% Hochsieder, der Rest besteht im wesentlichen aus o-TDA und m-TDA, wobei bevorzugt o-TDA überwiegt. Besonders bevorzugt enthält Strom P6 zwischen 30 und 70 Gew.-% Hochsieder und weniger als 10 Gew.-% m-TDA, der Rest ist im Wesentlichen o-TDA.

In einer ebenfalls bevorzugten Variante dieses Verfahrens wird man den aus dem Sumpf der Trennwandkolonne ausgeschleusten Produktstrom P4, der Hochsieder und m-TDA enthält, weiter verarbeiten, um die Verluste an m-TDA weiter zu verringern. Hierzu kann beispielsweise ein Knetertrockner eingesetzt werden. Dieser wird unter Hitze im Vakuum betrieben. Auf diese Weise wird das im Zulauf zum Knetertrockner enthaltene m-TDA verdampft. Den so erhaltenen Brüdenstrom gibt man beispielsweise auf einen Kondensator und erhält so m-TDA, das man in den Prozess zurückführt oder mit dem Produktstrom P3 mischen kann. In einer anderen Variante des erfindungsgemäßen Verfahrens kann man den Brüdenstrom direkt in die Trennwandkolonne zurückführen. Um die Viskosität des erhaltenen Hochsieders zu verringern, kann man diesen mit einer geeigneten niedrig-viskosen Flüssigkeit vermischen. Bevorzugt setzt man hierzu, sofern vorhanden, einen Leichtsieder enthaltenden Strom (beispielsweise Strom P5 in Figur 2) und / oder den o-TDA enthaltenden Produktstrom P2 ganz oder teilweise ein.

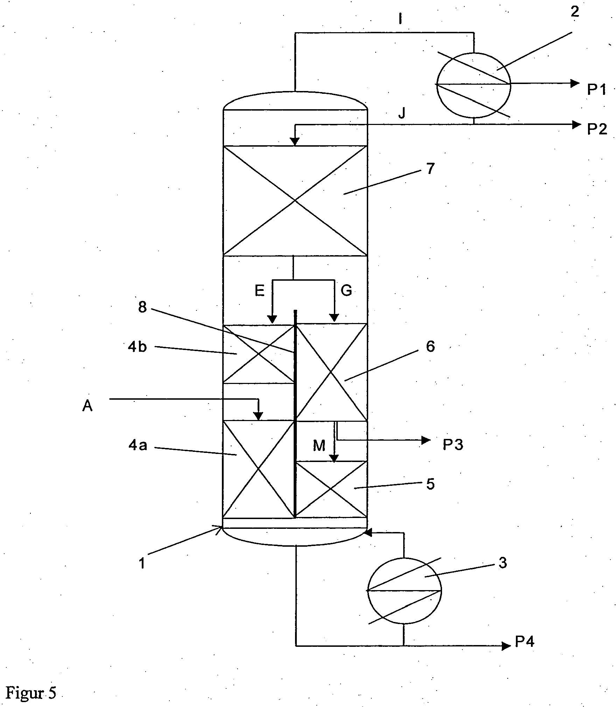

In einer weiteren Ausführungsform des erfindungsgemäßen Verfahrens ist das Trennsegment zur Vortrennung des Zulaufs A in ein oberes Teiltrennsegment 4b und ein darunter angeordnetes, unteres Teiltrennsegment 4a unterteilt. Diese bevorzugte Ausführungsform ist in **Figur 5** dargestellt. Der Zulauf A wird dabei zwischen dem oberen Teiltrennsegment 4b und dem unteren Teiltrennsegment 4a auf die Trennwandkolonne 1 aufgegeben. Diese Anordnung ist insbesondere dann vorteilhaft, wenn der Kolonnenzulauf A Hochsieder enthält, die nur wenig oberhalb von m-TDA sieden. In diesem Fall dient das Segment 4b zur Abtrennung dieser Hochsieder vom Zulauf und vermindert so den Gehalt an diesen Hochsiedern im Rücklauf E und G und somit auch in Produktstrom P3. Die Höhe der Energieeinsparung hängt dabei wesentlich vom o-TDA-Gehalt im Zulauf, dem geforderten o-TDA-Gehalt im Produkt P3, der für die Abtrennung der Hochsieder erforderlichen Zahl an theoretischen Trennstufen des Segmentes 5 sowie dem dort erforderlichen Rücklauf M ab. Daher muss die Auswahl und Auslegung der Variante im Einzelfall erfolgen. Die Zuführung des Roh-TDA (Strom A) erfolgt dabei seitlich von der Trennwand 8, wobei die Aufgabe des Zulaufs A von oben auf das Teiltrennsegment 4a erfolgt, dessen Oberkante mindestens 10%, bevorzugt mindestens 20% der Gesamthöhe der Trennwand 8 unterhalb der Oberkante der Trennwand 8 liegt und mindestens 10%, bevorzugt mindestens 30% der Gesamthöhe der Trennwand 8 oberhalb der Unterkante der Trennwand 8 liegt.

Es können alle in der Technik für diesen Zweck bekannten Verdampfer (z.B. Verdampfer 3 in den Figuren 1 bis 5) eingesetzt werden. So sind beispielsweise Naturumlaufverdampfer, Zwangsumlaufverdampfer oder Einsteckverdampfer geeignet. Bevorzugt wird man liegende Verdampfer vom Typ Kettle oder stehende Fallfilmverdampfer einsetzen. Ähnliches gilt für die Kondensatoren (z.B. Kondensator 2 in den Figuren 1, 3, 4, und 5 oder Kondensatoren 2 und 9 in Figur 2). Da die Trennwandkolonne infolge des hohen Siedepunktes des TDA im Vakuum betrieben wird, empfehlen sich Kondensatoren mit niedrigem Druckverlust. Bevorzugt kann der erste Kondensator (z.B. Kondensator 2 in Figur 2) in die Trennwandkolonne integriert und als Gleich- oder Gegenstromkondensatoren ausgeführt werden. In diesem Fall würde man die gegebenenfalls zusätzlich vorhandenen Kondensatoren (z.B. Kondensator 9 in Figur 2) neben der Trennwandkolonne anordnen. In einer anderen bevorzugten Ausführung des erfindungsgemäßen Verfahrens ist der Kondensator (z.B. Kondensator 2 in den Figuren 1 bis 5) und gegebenenfalls zusätzlich vorhandene Kondensatoren (z.B. Kondensator 9 in Figur 2) neben der Kolonne angeordnet.

Bezüglich der in der Destillationskolonne verwendeten Trennsegmente bestehen keine grundsätzlichen Einschränkungen. So können beispielsweise Füllkörperschüttungen und alle Typen von Böden eingesetzt werden, bevorzugt sind jedoch Glockenböden und besonders bevorzugt ungeordnete Füllkörperschüttungen oder geordnete Packungen mit niedrigem Druckverlust.

Die erfindungsgemäß eingesetzte Trennwandkolonne wird bei absoluten Kopfdrücken von 30 bis 500 mbar, bevorzugt bei 50 bis 300 mbar betrieben. Dabei stellen sich - abhängig von der Zusammensetzung des Zulaufs - Kopftemperaturen von 100 bis 200°C, bevorzugt 110 bis 190°C ein. Die Sumpftemperaturen betragen dabei bevorzugt 170 bis 300°C, besonders bevorzugt 180 bis 260°C. Die Zulauftemperatur beträgt bevorzugt 130 bis 250°C, besonders bevorzugt 150 bis 230°C.

Die Regelung der Trennwandkolonne in dem erfindungsgemäßen Verfahren kann unter Umständen schwierig sein, wobei jedoch die verschiedenen möglichen Vorgehensweisen grundsätzlich bekannt sind. Bevorzugt wird man im oberen Verstärkerteil der Trennwandkolonne, d.h. oberhalb der Trennwand 8, eine Temperaturregelung vorsehen, die als Stellgröße den Destillatstrom, das Rücklaufverhältnis oder bevorzugt die Rücklaufmenge J nutzt. Die Ausschleusung des Sumpfproduktstroms P4 kann beispielsweise über eine Flussregelung erfolgen, die einen bestimmten Prozentsatz des Kolonnenzulaufs A im Sumpf (P4) ausschleust. Dies bietet sich besonders dann an, wenn der Hochsiederanteil im Zulauf bekannt ist und nicht zu stark schwankt. In diesem Fall könnte man beispielsweise den Rücklauf M als Stellgröße für eine Standregelung im Sumpf der Kolonne oder im Verdampfer einsetzen. Hierfür würde man die Trennwandkolonne um einen an der Seitenentnahme angeordneten Rücklaufsammelbehälter ergänzen, aus dem der Rücklauf M auf den Abtriebsteil der Hauptsegments gegeben wird. Diesen Behälter könnte man beispielsweise mit einer Standregelung ausstatten, die auf die ausgeschleuste Menge des Produkts P3 als Stellgröße wirkt. Mit diesen Maßnahmen wird ein stabiler Betrieb der Kolonne gewährleistet und damit eine weiter verbesserte Produktreinheit erreicht.

Mit Hilfe des erfindungsgemäßen Verfahrens wird ein m-TDA erhalten, das bei der Phosgenierung nach den gängigen Verfahren nach dem Stand der Technik (z.B. Ullmann Encyclopedia of Industrial Chemistry, Wiley-VCH, 7th. Edition, Release 2005 unter dem Stichwort "Isocyanates, organic") zu besonders hohen Ausbeuten führt. Dabei wird ein m-TDI erhalten, das eine besonders geringe Färbung und eine besonders geringe Vergilbungsneigung besitzt.

### Beispiele

### Beispiel 1

Ein Strom A von 6931 kg/h, bestehend neben m-TDA aus 39 kg/h p-TDA, 251,8 kg/h o-TDA, 25,8 kg/h Leichtsiedern, 90,8 kg/h Hochsiedern wird auf eine Trennwandkolonne 1 gegeben. Die Kolonne ist wie in Fig.5 dargestellt aufgebaut. Die Zahl der theoretischen Böden der einzelnen Trennsegmente beträgt 17 Böden für Trennsegment 4a, 9 Böden für Trennsegment 5, 17 Böden für Trennsegment 6, 22 Böden für Trennsegment 7 und 9 Böden für Trennsegment 4b. Die Trennwandkolonne wird bei einem absoluten Kopfdruck von 90 mbar, gemessen hinter dem Kondensator 2 betrieben. Die Menge an Rücklauf J beträgt 9210 kg/h, die Leistung des Verdampfers 3 beträgt 1446 kW. Das Verhältnis der beiden Ströme E zu G wird zu 0,2 zu 0,8 eingestellt. Als Strom M werden 1487,8 kg/h auf das Trennsegment 5 aufgegeben.

Unter diesen Bedingungen erhält man einen Strom P4 von 166,5 kg/h, der 45,3 Gew.-% m-TDA enthält. Als Produktstrom P3 werden 6491 kg/h ausgeschleust, wobei der Strom P3 neben m-TDA 0,59 Gew.-% p-TDA und 0,10 Gew.-% o-TDA enthält. Der Produktstrom P2 beträgt 158,9 kg/h und enthält 3,2 Gew.-% Leichtsieder und 1,0 Gew.% m-TDA. Strom P1 enthält 18 Gew.-% Leichtsieder und ansonsten im wesentlichen o-TDA.

### Vergleichsbeispiel 1:

Dieses Beispiel wird gemäß dem Stand der Technik durchgeführt. Der in Beispiel 1 definierte Strom A wird auf eine konventionelle Isomerentrennkolonne aufgegeben. Das unterhalb des Zulaufs befindliche Segment hat dabei 17 theoretische Trennstufen, das oberhalb des Zulaufs befindliche Segment hat 30 theoretische Trennstufen. Das aus dem Sumpf der Isomerentrennkolonne ausgeschleuste Gemisch aus m-TDA und Hochsiedern wird in einem nachgeschalteten Verdampfer teilweise verdampft. Aus dem Verdampfer wird ein flüssiger Strom ausgeschleust der aus Hochsiedern und m-TDA besteht. Der aus dem Verdampfer erhaltene Brüdenstrom wird kondensiert und stellt das von Hochsiedern befreite m-TDA dar.

Die Isomerentrennkolonne wird bei einem Kopfdruck von 90 mbar, gemessen nach dem Kondensator betrieben. Der Rücklauf beträgt 8876 kg/h, die Leistung des Verdampfers der Isomerentrennkolonne beträgt 1392 kW.

Unter diesen Bedingungen werden als flüssiges Kopfprodukt ein Strom von 199 kg/h ausgeschleust, dieser Strom enthält 1,0% m-TDA und 4,3% Leichtsieder. Das nicht kondensierte gasförmige Kopfprodukt enthält dabei 23% Leichtsieder. Die nachgeschaltete Verdampfer und Kondensator wird bei einem Druck von 150 mbar betrieben, die Temperatur im Austritt des Verdampfers beträgt 231°C. Im Sumpf werden 160 kg/h ausgeschleust, der m-TDA-Gehalt dieses Stroms (P4) beträgt 49%. Der aus dem Kondensator als Produkt entnommene Strom beträgt 6497 kg/h, der o-TDA-Gehalt 0,1%, der p-TDA-Gehalt 0,59%, der Hochsiedergehalt 0,2%. Der Verdampfer wird mit einer Heizleistung von 1039 kW betrieben.

Man erkennt, dass für die nach dem Stand der Technik durchgeführte Trennung von 6931 kg/h Roh-TDA ein Energieaufwand von 2431 KW erforderlich ist. Nach dem erfindungsgemäßen Verfahren sind nur 1446 KW erforderlich.

## Patentansprüche

1. Verfahren zur Herstellung von m-Toluylendiamin, bei dem
a) Dinitrotoluol in Gegenwart eines Katalysators hydriert und das erhaltene Reaktionsgemisch von Katalysator, Wasser und gegebenenfalls Lösungsmittel getrennt wird, wobei Roh-Toluylendiamin erhalten wird, und
b) das Roh-Toluylendiamin in einer Trennwandkolonne destillativ getrennt wird, wobei wenigstens vier Produktströme P1 - P4 erhalten werden, wobei
der Produktstrom P1 ein Leichtsieder enthaltender Strom ist, und
der Produktstrom P2 ein o-Toluylendiamin enthaltender Strom ist, und
der Produktstrom P3 ein m-Toluylendiamin enthaltender Strom ist, und
der Produktstrom P4 ein Hochsieder und m-Toluylendiamin enthaltender Strom ist, und bei dem das Roh-Toluylendiamin der Trennwandkolonne seitlich von der Trennwand (8) zugeführt wird, wobei die Aufgabe des Roh-Toluylendiamin von oben auf ein Trennsegment (4a) erfolgt, dessen Oberkante mindestens 10 % der Gesamthöhe der Trennwand unterhalb der Oberkante der Trennwand liegt und mindestens 10 % der Gesamthöhe der Trennwand oberhalb der Unterkante der Trennwand liegt.

2. Verfahren nach Anspruch 1, bei dem der Hochsieder und m-Toluylendiamin enthaltende Strom P4 aus dem Sumpf der Trennwandkolonne entnommen wird.

3. Verfahren nach einem der Ansprüche 1 bis 2, bei dem der m-Toluylendiamin enthaltende Strom P3 der Trennwandkolonne im Bereich der Trennwand entnommen wird.

4. Verfahren nach einem der Ansprüche 1 bis 2, bei dem der o-Toluylendiamin enthaltende Produktstrom P2 der Trennwandkolonne als Seitenstrom entnommen wird.

5. Verfahren nach einem der Ansprüche 1 bis 3, bei dem der am Kopf der Trennwandkolonne entnommene Brüdenstrom kondensiert wird und das Kondensat teilweise als o-Toluylendiamin enthaltender Produktstrom P2 entnommen wird und teilweise als Rücklauf auf die Trennwandkolonne aufgegeben wird.

6. Verfahren nach Anspruch 5, bei dem der Brüdenstrom zunächst nur teilweise kondensiert wird, und das Kondensat teilweise als o-Toluylendiamin enthaltender Produktstrom P2 entnommen wird und teilweise als Rücklauf auf die Trennwandkolonnen aufgegeben wird.

7. Verfahren nach einem der Ansprüche 1 bis 6, bei dem der Hochsieder und m-Toluylendiamin enthaltende Produktstrom P4 der Trennwandkolonne aus dem Sumpf entnommen wird und anschließend in einem zusätzlichen Apparat m-Toluylendiamin abgetrennt wird.

8. Verfahren nach Anspruch 7, bei dem, die Abtrennung des m-Toluylendiamin durch Verdampfung in einem Knetertrockner erfolgt.

9. Verfahren nach Anspruch 7, bei dem der Produktstrom P4 einer Destillationskolonne (11) am Kopf zugeführt wird, und der o-Toluylendiamin enthaltende Produktstrom P2 ganz oder teilweise der Destillationskolonne (11) unterhalb des Zulaufs des Produktstroms P4 zugeführt wird, und aus der Destillationskolonne (11) ein Strom S entnommen und in die Trennwandkolonne (1) zurückgeführt wird.

## Claims

1. Process for the preparation of m-tolylenediamine, wherein
a) dinitrotoluene is hydrogenated in the presence of a catalyst and the resulting reaction mixture is separated from the catalyst, water and optionally solvent to give crude tolylenediamine, and
b) the crude tolylenediamine is separated by distillation in a dividing wall column to give at least four product streams P1 - P4, where
the product stream P1 is a stream containing low boilers,
the product stream P2 is a stream containing o-tolylenediamine,
the product stream P3 is a stream containing m-tolylenediamine,
the product stream P4 is a stream containing high boilers and m-tolylenediamine, and
wherein the crude tolylenediamine is introduced into the dividing wall column at the side of the dividing wall (8), the crude tolylenediamine being introduced from above into a separating segment (4a), the upper edge of which is at least 10% of the total height of the dividing wall below the upper edge of the dividing wall and at least 10% of the total height of the dividing wall above the lower edge of the dividing wall.

2. Process according to Claim 1 wherein the stream P4 containing high boilers and m-tolylenediamine is withdrawn from the bottom of the dividing wall column.

3. Process according to one of Claims 1 to 2 wherein the stream P3 containing m-tolylenediamine is withdrawn from the dividing wall column in the region of the dividing wall.

4. Process according to one of Claims 1 to 2 wherein the product stream P2 containing o-tolylenediamine is withdrawn from the dividing wall column as a side stream.

5. Process according to one of Claims 1 to 3 wherein the vapour stream withdrawn from the top of the dividing wall column is condensed and part of the condensate is withdrawn as the product stream P2 containing o-tolylenediamine and part is introduced into the dividing wall column as reflux.

6. Process according to Claim 5 wherein initially only part of the vapour stream is condensed and part of the condensate is withdrawn as the product stream P2 containing o-tolylenediamine and part is introduced into the dividing wall columns as reflux.

7. Process according to one of Claims 1 to 6 wherein the product stream P4 containing high boilers and m-tolylenediamine is withdrawn from the bottom of the dividing wall column and m-tolylenediamine is then separated off in an additional apparatus.

8. Process according to Claim 7 wherein the m-tolylenediamine is separated off by evaporation in a kneader dryer.

9. Process according to Claim 7 wherein the product stream P4 is introduced into the top of a distillation column (11), all or part of the product stream P2 containing o-tolylenediamine is introduced into the distillation column (11) below the inflow of the product stream P4, and a stream S is withdrawn from the distillation column (11) and recycled into the dividing wall column (1).

## Revendications

1. Procédé de fabrication de m-toluylène-diamine, selon lequel
a) du dinitrotoluène est hydrogéné en présence d'un catalyseur, et le mélange réactionnel obtenu est séparé du catalyseur, de l'eau et éventuellement du solvant, de la toluylène- diamine brute étant obtenue, et
b) la toluylène-diamine brute est séparée par distillation dans une colonne à paroi de séparation, au moins quatre courants de produits P1 à P4 étant obtenus,
le courant de produits P1 étant un courant contenant des composants de point d'ébullition faible, et
le courant de produits P2 étant un courant contenant de l'o-toluylène-diamine, et le courant de produits P3 étant un courant contenant de la m-toluylène-diamine, et le courant de produits P4 étant un courant contenant des composants de point d'ébullition élevé et de la m-toluylène-diamine, et
selon lequel la toluylène-diamine brute est introduite dans la colonne à paroi de séparation sur le côté de la paroi de séparation (8), l'introduction de la toluylène-diamine brute ayant lieu par le haut sur un segment de séparation (4a), dont le bord supérieur se situe au moins 10 % de la hauteur totale de la paroi de séparation sous le bord supérieur de la paroi de séparation et au moins 10 % de la hauteur totale de la paroi de séparation au-dessus du bord inférieur de la paroi de séparation.

2. Procédé selon la revendication 1, selon lequel le courant P4 contenant des composants de point d'ébullition élevé et de la m-toluylène-diamine est soutiré au fond de la colonne à paroi de séparation.

3. Procédé selon l'une quelconque des revendications 1 ou 2, selon lequel le courant P3 contenant de la m-toluylène-diamine est soutiré de la colonne à paroi de séparation dans la zone de la paroi de séparation.

4. Procédé selon l'une quelconque des revendications 1 à 2, selon lequel le courant de produits P2 contenant de l'o-toluylène-diamine est soutiré de la colonne à paroi de séparation sous la forme d'un courant latéral.

5. Procédé selon l'une quelconque des revendications 1 à 3, selon lequel le courant de vapeur soutiré à la tête de la colonne à paroi de séparation est condensé et le condensat est soutiré en partie en tant que courant de produits P2 contenant de l'o-toluylène-diamine et introduit en partie en tant que reflux dans la colonne à paroi de séparation.

6. Procédé selon la revendication 5, selon lequel le courant de vapeur n'est tout d'abord condensé qu'en partie, et le condensat est soutiré en partie en tant que courant de produits P2 contenant de l'o-toluylène-diamine et introduit en partie en tant que reflux dans la colonne à paroi de séparation.

7. Procédé selon l'une quelconque des revendications 1 à 6, selon lequel le courant de produits P4 contenant des composants de point d'ébullition élevé et de la m-toluylène-diamine est soutiré de la colonne à paroi de séparation au fond, puis la m-toluylène-diamine est séparée dans un appareil supplémentaire.

8. Procédé selon la revendication 7, selon lequel la séparation de la m-toluylène-diamine a lieu par évaporation dans un séchoir-malaxeur.

9. Procédé selon la revendication 7, selon lequel le courant de produits P4 est introduit à la tête d'une colonne de distillation (11), et le courant de produits P2 contenant de l'o-toluylène-diamine est introduit en totalité ou en partie dans la colonne de distillation (11) en dessous de l'alimentation du courant de produits P4, et un courant S est soutiré de la colonne de distillation (11) et recyclé dans la colonne à paroi de séparation (1).
